# EUROPEAN PATENT APPLICATION

(11) **EP 4 671 362 A1**
(43) Date of publication of application: **31.12.2025**
(21) Application number: 24760406.9
(22) Date of filing: 21.02.2024
(51) Int. Cl.: C12N 5/071

(54) **TRANSPARENTIZING POLYMER ENABLING DEEP OBSERVATION OF LIVING THREE-DIMENSIONAL TISSUE**

(30) Priority: 22.02.2023 JP 2023026167
(71) Applicant: The University of Osaka, Osaka 565-0871 (JP)
(72) Inventor: MATSUSAKI, Michiya, Suita-shi, Osaka 565-0871 (JP)
(74) Representative: Hoffmann Eitle
(86) International application number: PCT/JP2024/006257
(87) International publication number: WO 2024/177108

(57) **Abstract**

Provided are: a compound containing a soluble polysaccharide or a derivative thereof and a nucleotide or a derivative thereof; a method for making living biological tissue transparent using said compound, and a kit and a composition for said method; and a method for making living biological tissue transparent using dextrin or a derivative thereof, and a kit and a composition for said method.

## Description

### Technical Field

The present invention relates to a polymer capable of making biological tissue transparent in a living state, a method for making biological tissue transparent using the polymer, a composition and a kit for the method, and the like. The present application claims priority to Japanese Patent Application No. 2023-26167, the entirety of which is incorporated herein by reference.

### Background Art

In medical and drug discovery studies, it is important to accurately observe a three-dimensional structure of biological tissue, organs, or three-dimensional tissue models with a confocal laser microscope or the like. However, biological tissue is usually opaque and less light-transmissive, and therefore, the observation depth is limited to 100 to 200 µm. As a result, various transparentizing methods have been reported in recent years. Many of them are approaches that increase the light-transmitting property by replacing water with organic molecules with a high refractive index so that it matches the refractive index of biomolecules (see Patent Literature 1). In addition, methods have also been reported in which the refractive index of tissue is reduced by removing lipids with a high refractive index (see Patent Literature 2).

However, since cells are dead after the treatment, it is necessary to fix the tissue with formaldehyde or the like, and no method has been reported for achieving making the cells transparent in a living state. Also, the cell structure collapses after the lipids (cell membranes) are removed, making it impossible to observe tiny molecules such as membrane proteins.

The present inventors' group has succeeded in making tissue transparent in a living state and observing deep tissue by applying certain nucleic acid constituents to the tissue (see Patent Literature 3). However, there has been a demand for the development of a transparentizing agent whose cytotoxicity is further reduced.

### Citation List

### Patent Literature

Patent Literature 1: International Patent Application No. WO 2015/022883
Patent Literature 2: International Patent Application No. WO 2017/188264
Patent Literature 3: International Patent Application No. WO 2021/060373

### Summary of Invention

### Technical Problem

There has been a demand for discovering an agent capable of making tissue transparent while keeping it alive for a long period of time.

### Solution to Problem

The present inventors have conducted intensive research and found that tissue can be made transparent while keeping it alive for a long period of time, by using a compound containing a soluble polysaccharide or a derivative thereof, and a nucleotide or a derivative thereof. Further, the present inventors have found that biological tissue can be made transparent in a similar manner when dextran is used alone. The present inventors have completed the present invention based on these findings.

In other words, the present invention provides the following.
(1) A compound comprising a soluble polysaccharide or a derivative thereof, and a nucleotide or a derivative thereof.
(2) The compound according to (1), in which the soluble polysaccharide is selected from the group consisting of dextran, hyaluronic acid, alginic acid, gellan gum, and maltodextrin, and the nucleotide is selected from the group consisting of AMP, CMP, TMP, and ADP.
(3) The compound according to (1) or (2), in which the soluble polysaccharide is dextran.
(4) A method for making biological tissue transparent in a living state, comprising applying a solution comprising the compound according to any of (1) to (3) to the biological tissue.
(5) A composition for making biological tissue transparent in a living state, comprising the compound according to any of (1) to (3).
(6) A kit for making biological tissue transparent in a living state, comprising the compound according to any of (1) to (3).
(7) A method for observing biological tissue, comprising making the biological tissue transparent in a living state by using the compound according to any of (1) to (3), the method according to (4), the composition according to (5), or the kit according to (6), and observing the inside of the biological tissue.
(8) A method for producing biological tissue made transparent in a living state, comprising applying the compound according to any of (1) to (3), the method according to (4), the composition according to (5), or the kit according to (6) to the biological tissue.
(9) A method for making biological tissue transparent in a living state, comprising applying a solution comprising dextran or a derivative thereof to the biological tissue.
(10) The solution according to (9), in which the dextran or a derivative thereof is present at a concentration such that the refractive index of the solution is 1.35 or more.
(11) A composition for making biological tissue transparent in a living state, comprising dextran or a derivative thereof.
(12) A kit for making biological tissue transparent in a living state, comprising dextran or a derivative thereof.
(13) A method for observing biological tissue, comprising making the biological tissue transparent in a living state by using dextran or a derivative thereof, the method according to (9) or (10), the composition according to (11), or the kit according to (12), and observing the inside of the biological tissue.
(14) A method for producing biological tissue made transparent in a living state, comprising applying the dextran or a derivative thereof, the method according to (9) or (10), the composition according to (11), or the kit according to (12) to the biological tissue.

### Advantageous Effects of Invention

According to the present invention, biological tissue can be made transparent in a living state for a long period of time, and therefore, the state of deep biological tissue can be observed as it is. The state of blood vessels and lymphatic vessels of the deep biological tissue, gene expression, localization of substances, and the like can be observed over time. In addition, since it is possible to observe not only the surface of a graft but also its deep part, the evaluation of the graft can be accurately conducted. Therefore, it can be deemed that the significance of the ability of making biological tissue transparent in a living state is extremely important in the development of pharmaceuticals, medical and pharmaceutical research, and the like. Further, commercially available products can be used as materials to prepare the compound of the present invention, leading to reduction of the cost of the compound of the present invention.

### Brief Description of Drawings

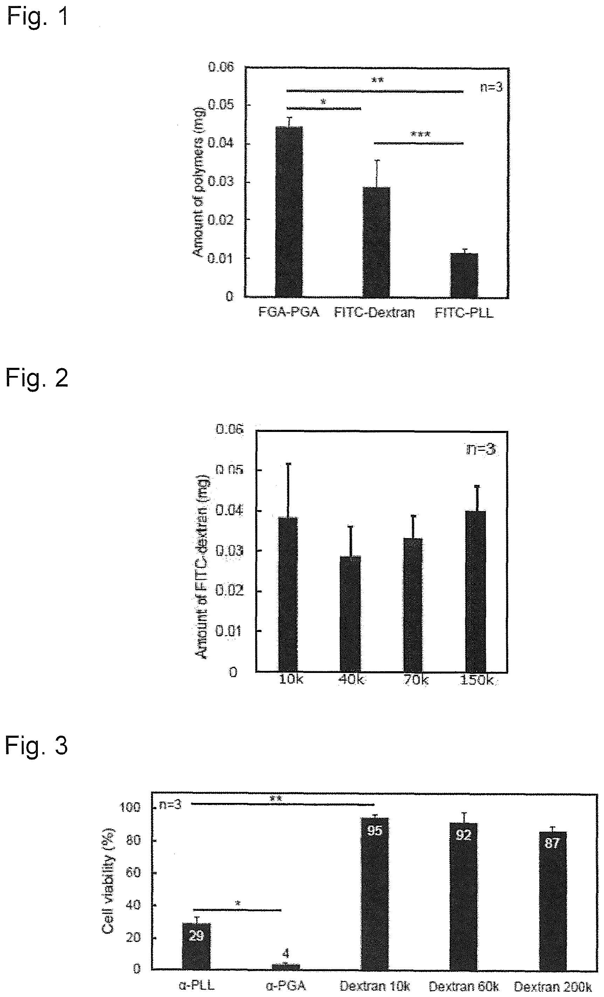

[Figure 1] Figure 1 shows the results of investigating the permeability of FITC-α-polylysine (FITC-PLL) (15 kDa - 30 kDa), FITC-dextran (40 kDa), and FGA-α-polyglutamic acid (FGA-PGA) (15 kDa - 30 kDa) into collagen gels. One-star indicates p<0.05, two-star indicates p<0.01, and three-star indicates p<0.005.
[Figure 2] Figure 2 shows the results of investigating the permeability of FITC-dextran (10 kDa, 40 kDa, 70 kDa, and 150 kDa) into collagen gels.
[Figure 3] Figure 3 shows the results of investigating the viability of cells to which PLL (1 kDa - 5 kDa), dextran (10 kDa, 60 kDa, and 200 kDa), and PGG (15 kDa to 50 kDa) were applied. One-star indicates p<0.05, and two-star indicates p<0.01.

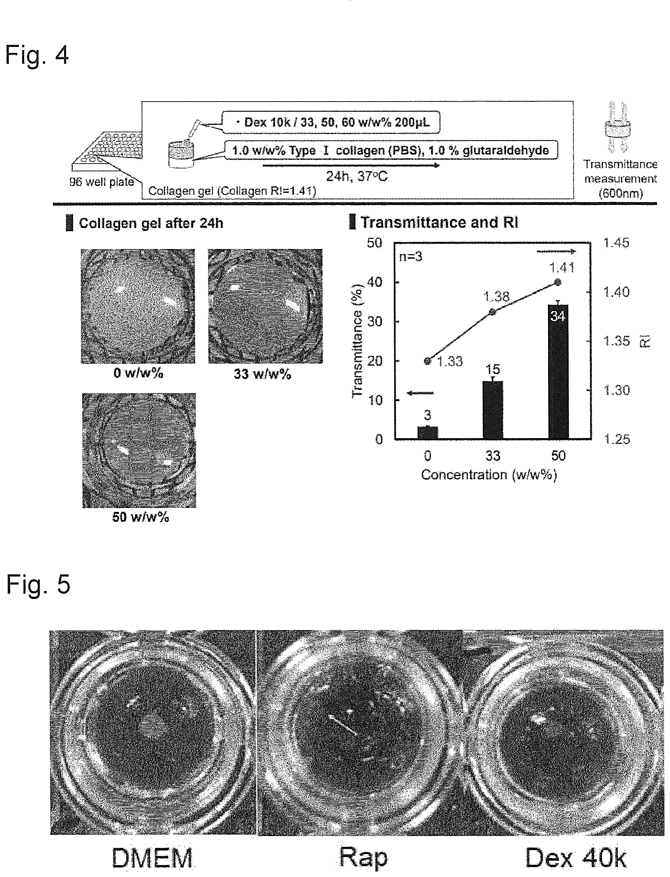

[Figure 4] Figure 4 shows the results of investigating the impact of the dextran concentrations on the transparency of collagen gels. The upper panel indicates an outline of the experimental procedure. The lower left panel is photos of collagen gels after incubation with dextran at various concentrations. The lower right panel is a graph showing the relationship between dextran concentrations and light transmittance, and the refractive index of the dextran solution.
[Figure 5] Figure 5 shows the results of transparency experiment with dextran on spheroids prepared using mouse L929 cells. DMEM is a spheroid added with DMEM (transparent medium), Rap is a spheroid added with RapiClear, (an arrow indicates the location of the spheroid), and Dex 40k is a spheroid added with dextran.

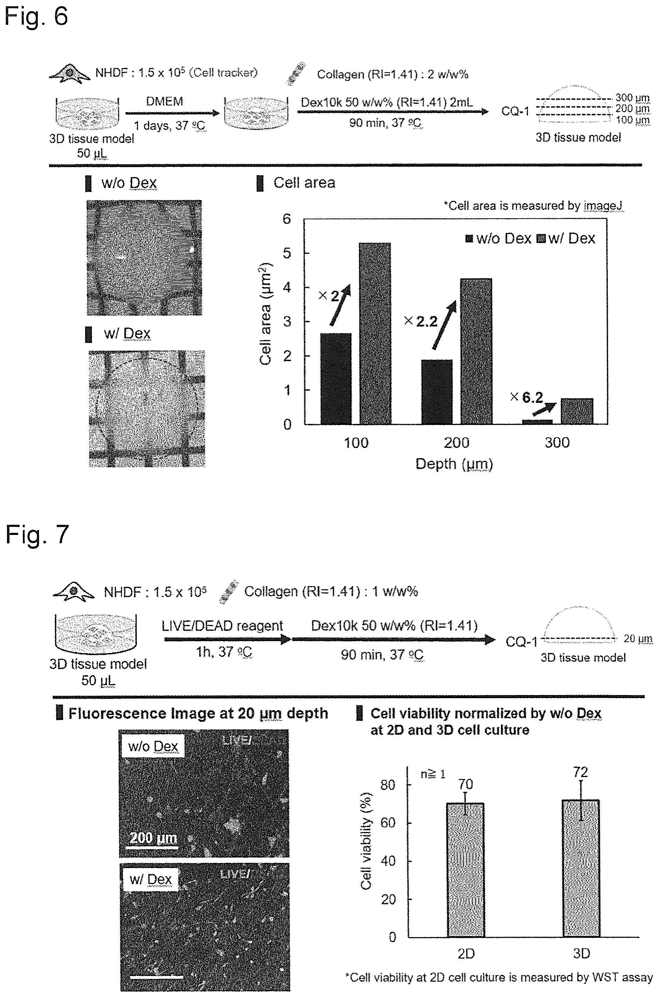

[Figure 6] Figure 6 shows the results of performing deep observation of three-dimensional tissue using dextran. The upper panel indicates an outline of the experimental procedure. The lower left panel is photos of tissue with dextran added (w/ DEX) and without dextran added (w/o DEX) after incubation. The lower right panel is a graph showing the results of performing observation of deep tissue (100, 200, and 300 µm from the bottom) with a confocal quantitative image cytometer and measuring the area where cells are visible (with dextran added (w/ DEX) and without dextran added (w/o DEX)).
[Figure 7] Figure 7 shows the results of investigating the impact of dextran on the viability of cells in the three-dimensional tissue. The upper panel indicates an outline of the experimental procedure. The lower left panel shows LIVE/DEAD stained images of deep three dimensional tissue (20 µm from the bottom). The lower right panel is a graph showing the cell viability after incubation. The numerical values in the graph are the viability when the viability without dextran added is set to 100. 3D indicates three-dimensional tissue, and 2D indicates two-dimensional tissue.

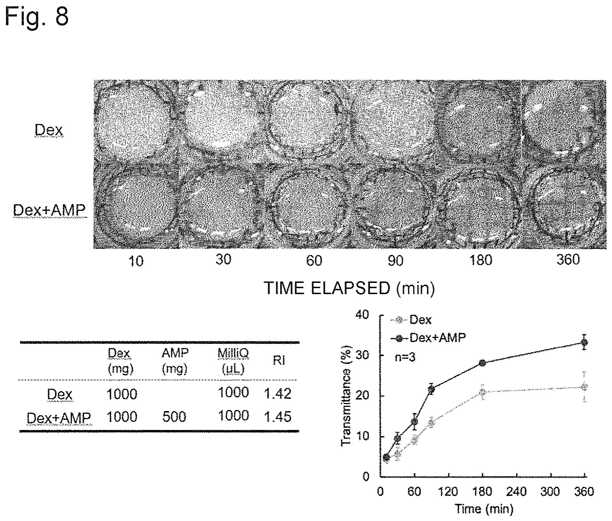

[Figure 8] Figure 8 shows the combined effect of dextran and AMP. Dex indicates a dextran solution, and Dex + AMP indicates a dextran solution with AMP added. The upper panel is a photo showing the progress in transparency over time. The lower left panel shows the composition of the solution used in the experiment. RI is the refractive index of the solution. The lower right panel is a graph showing the change in transmittance of the samples over time.

### Description of Embodiments

In one aspect, the present invention provides a compound containing a soluble polysaccharide or a derivative thereof, and a nucleotide or a derivative thereof (hereinafter, referred to as the "compound of the present invention").

In the compound of the present invention, a nucleotide or a derivative thereof is bound to a soluble polysaccharide or a derivative thereof. Other compounds can be bound to the soluble polysaccharide or a derivative thereof, and/or the nucleotide or a derivative thereof in the compound of the present invention. The type of bond between the soluble polysaccharide or a derivative thereof, and the nucleotide or a derivative thereof can be any type, and is typically a covalent bond.

The soluble polysaccharide is a polysaccharide that can be dissolved in water or a medium containing water. Any type of soluble polysaccharide may be used. The soluble polysaccharide may be naturally derived or artificially prepared. Preferred soluble polysaccharides are those having compatibility with a living body, those having low or no toxicity, and those capable of permeating into biological tissue. Preferred examples of the soluble polysaccharide include, but not limited to, dextran, hyaluronic acid, alginic acid, gellan gum, and maltodextrin. More preferred examples of the soluble polysaccharide include, but not limited to, dextran. In addition, soluble polysaccharides that are neutral or close to neutral in charge are preferable. Dextran is a known soluble polysaccharide that is composed only of glucose and contains many α-1,6 glycosidic bonds.

Derivatives of soluble polysaccharides are well-known. Examples thereof include, but not limited to polysaccharides in which the hydroxyl groups have been alkylated or esterified (e.g., methylated polysaccharide), polysaccharides in which the hydroxyl groups have been substituted with other groups such as a carboxyl group, O-glycosides, and N-glycosides. Derivatives of the soluble polysaccharides can be obtained using known methods and materials.

The molecular weight of the soluble polysaccharide or a derivative thereof is not particularly limited. For example, in the case of dextran, the molecular weight may be 10 kDa or more, such as 20 kDa or more, 40 kDa or more 60 kDa or more, 100 kDa or more, 150 kDa or more, or 200 kDa or more, and may be, for example, 10 kDa - 40 kDa, 10 kDa - 60 kDa, 10 kDa - 100 kDa, 10 kDa - 150 kDa, 10 kDa - 200 kDa, 20 kDa - 60 kDa, 20 kDa - 100 kDa, 20 kDa - 150 kDa, 20 kDa - 200 kDa, 40 kDa - 100 kDa, 40 kDa - 150 kDa, 40 kDa - 200 kDa, 60 kDa - 150 kDa, 60 kDa - 200 kDa, and 100 kDa - 200 kDa. Dextran of various molecular weights are commercially available.

Since the soluble polysaccharides such as dextran or their derivatives are polymeric, their osmotic pressures are low even when used at a higher concentration. Further, the soluble polysaccharides or their derivatives have permeability to biological tissue. On the other hand, since the nucleotides such as AMP or their derivatives are highly capable of making tissue transparent but low molecular weight, their osmotic pressures are high when used at a higher concentration, causing damage to biological tissue. In the compound of the present invention, since a nucleotide or a derivative thereof is bound to a soluble polysaccharide or a derivative thereof, the refractive index per unit polymer increases. Accordingly, a higher refractive index can be achieved at a lower concentration, preventing the osmotic pressure from increasing. Therefore, the compound of the present invention causes less damage to biological tissue while retaining the transparentizing ability of nucleotides or their derivatives.

Nucleotides are known. Any type of nucleotide may be used. Preferred examples of the nucleotide include, but not limited to, AMP, CMP, TMP, and ADP. More preferred examples of the nucleotide include, but not limited to, AMP and ADP. Further preferred examples of the nucleotide include, but not limited to, AMP.

Derivatives of the nucleotides are well-known and can be obtained using known methods and materials. Derivatives of the nucleotides may be formed at any of the sugar, base, or phosphate group. For example, the hydroxyl group of the sugar may be alkylated, esterified, acetylated, or the like. In addition, the amino group of the base may be acylated, amidated, or the like. Glycosides can be formed in the sugar moiety. The hydroxyl group of the phosphate may be substituted. Examples of the derivatives of the nucleotide are not limited to the above examples.

The compound of the present invention can be obtained by binding a soluble polysaccharide or a derivative thereof and a nucleotide or a derivative thereof by a known method. The bond may be any type, and is typically a covalent bond. For example, a soluble polysaccharide or a derivative thereof and a nucleotide or a derivative thereof can be covalently bound by a method such as condensation reaction, nucleophilic substitution reaction, thiol-ene reaction, or click reaction. The ratio of the soluble polysaccharide or a derivative thereof to the nucleotide or a derivative thereof to be bound is not particularly limited. For example, it may be 0.1 mol% to 99 mol%, and it is desirably 1 mol% to 90 mol%, and more desirably 5 mol% to 80 mol%.

The compound of the present invention can contain one type of soluble polysaccharide or a derivative thereof, or two or more kind. The compound of the present invention can contain one type of nucleotide or a derivative thereof, or two or more kind. For example, in the compound of the present invention, AMP and ADP may be bound to dextran.

In a further aspect, the present invention provides a method for making biological tissue transparent in a living state, comprising applying the compound of the present invention to biological tissue.

In the method of the present invention, a solution comprising the compound of the present invention is applied to biological tissue. The solution can be made by dissolving the compound of the present invention in water, a buffer solution, a medium, or the like. The solution can contain a known transparentizing agent such as RapiClear. Buffer solutions are known and can be selected appropriately. Examples of the buffer solution include, but not limited to, Tris-HCl buffer solution, phosphate buffer solution, phosphate buffer physiological saline, and carbonate buffer solution.

The concentration of the compound of the present invention in the solution is preferably such that the refractive index of the solution is about 1.35 or more, and preferably about 1.4 or more. Since the refractive index varies depending on the compound, the concentration required to obtain a desired refractive index also varies depending on the compound. The selection of the compound and the adjustment of its concentration are within the scope of those skilled in the art. Means and methods for measuring the refractive index are known.

The above solution may be applied to the biological tissue in any manner as long as the compound of the present invention can surround and permeate the biological tissue, and make the biological tissue transparent. The above solution can be poured into a container to which the biological tissue is attached so that the entire tissue is immersed, or the biological tissue can be suspended in the above solution in a suitable container. In one specific example, a medium is removed from the biological tissue cultured in a dish, a well, on a membrane, or the like, and then the above solution is added to cover the biological tissue. The tissue is left still until a desired degree of transparency is obtained, and then the above solution is removed to observe the tissue. In another specific example, the biological tissue is isolated from a culture medium and transferred to a container containing the above solution. The tissue is left still until a desired degree of transparency is obtained, and then the above solution is removed to observe the tissue. The applying manner is not limited to the specific examples above.

The biological tissue encompasses any tissue derived from any organism, and examples thereof include, but not limited to, those derived from animals, plants, fish, amphibians, reptiles, insects, and microorganisms. The biological tissue may be derived from, for example, mammals such as humans, monkeys, dogs, cats, pigs, cows, rabbits, rats, mice, and guinea pigs, or from birds such as chickens.

The biological tissue may be derived from any part of an organism. When the organism is an animal, the biological tissue may be derived from, for example, the heart, lungs, liver, kidneys, stomach, intestines, pancreas, gallbladder, reproductive organs, brain, skin, and muscles. The biological tissue may be biopsy tissue, tissue obtained by surgery, or tissue obtained by dissection. The biological tissue may also be artificially produced. Examples of the artificial biological tissue include, but not limited to, those produced by the LbL method described in Japanese Patent Laid-Open No. 2012-115254 or the like. The biological tissue may be obtained by a known culture method. The biological tissue may be a spheroid. The biological tissue may be a graft. The biological tissue may be normal tissue, or tissue having a disease, such as cancer tissue. Note that, the term biological tissue used herein encompasses cells, cell masses, tissue, organs, visceral organs, and individual organisms (excluding living human individuals). In addition, the biological tissue may be decellularized cells.

The living state refers to, for example, a state in which the viability of cells in the biological tissue that has been subjected to transparentizing treatment for 90 minutes using the compound of the present invention is about 50% or more, preferably about 60% or more, more preferably about 70% or more, further preferably about 80% or more, and most preferably about 90% or more. The viability of cells can be measured by a known method such as trypan blue staining.

When performing transparentizing, it may be performed so that the light transmittance at 600 nm at a desired site in the tissue is, for example, about 30% or more, preferably about 40% or more, more preferably about 50% or more, further preferably about 60% or more, and most preferably about 70% or more, for example, about 80% or more. Note that the transmittance is expressed as the ratio of the transmitted light to incident light. Means and methods for measuring the transmittance are known to those skilled in the art. Those skilled in the art can select and determine the conditions for achieving transparency, such as the selection of compound, concentration of compound, and time, temperature, pH, the presence or absence of agitation for transparentizing treatment, and the like, in consideration of the type of biological tissue, a desired transparency depth, and the like.

In a further aspect, the present invention provides a composition for making biological tissue transparent in a living state, comprising the compound of the present invention.

The form of the composition of the present invention is not particularly limited, and is usually a solution comprising the compound of the present invention. The solution is preferably a solution in which the compound of the present invention is dissolved in an aqueous medium. Examples of the aqueous medium include water and a buffer solution. The buffer solution is as described above. The concentration of the compound of the present invention in the solution is as described above. Alternatively, the composition of the present invention may be in a solid form (e.g., powders, granules, or pellets), or a semi-solid form (e.g., paste, or gel). The composition of the present invention can be diluted with an aqueous medium or dissolved in an aqueous medium before use. The composition of the present invention may further contain any compound, for example, any constituent of organic or inorganic substances. By applying the composition of the present invention to biological tissue, the biological tissue can be made transparent in a living state. The application is as described above.

The present invention provides a kit for making biological tissue transparent in a living state, comprising the compound of the present invention.

The kit of the present invention comprises the compound of the present invention as an essential constituent. Specifically, the kit of the present invention may comprise a container containing the compound of the present invention as a solution in an aqueous medium, or a container containing the compound of the present invention as, for example, powders, granules, pellets, past, or gel. The form of the container is not particularly limited, and may be, for example, a bottle, a bag, or a tube. Usually, an instruction manual is attached to the kit of the present invention.

In another aspect, the present invention provides a method for observing biological tissue, comprising making the biological tissue transparent in a living state by using the method, the composition, or the kit of the present invention described above, and observing the inside of the biological tissue.

In the observation method of the present invention, the biological tissue that has been made transparent in a living state obtained by using the method, the composition, or the kit of the present invention, is observed with the naked eye or equipment such as a microscope. Means and method for observation are known to those skilled in the art, and they can be appropriately selected and used according to the type and size of the tissue. For example, a transparent sample may be immunostained with a fluorescent-labeled antibody, and then a specific substance or site in the biological tissue may be detected with a fluorescence microscope. According to the observation method of the present invention, it is possible to observe the state of the deep part of the living biological tissue, such as the development of the vasculature, production of substances, gene expression, and lesions.

In yet another aspect, the present invention provides a method for producing biological tissue made transparent in a living state, comprising applying the method, the composition, or the kit of the present invention described above to the biological tissue.

As described above, the method, the composition, and the kit of the present invention can be preferably applied to the living biological tissue, but needless to say, they are also applicable to non-living biological tissue.

The present inventors have also found that dextran can make tissue transparent while keeping it alive. In other words, it is possible to make biological tissue transparent in a living state, using dextran or a derivative thereof. Accordingly, in a further aspect, the present invention provides a method for making biological tissue transparent in a living state, comprising applying a solution comprising dextran or a derivative thereof to the biological tissue.

Derivatives of dextran are well-known. Examples thereof include, but not limited to dextran in which the hydroxyl groups have been alkylated or esterified (e.g., methylated dextran), dextran in which the hydroxyl groups have been substituted with other groups such as a carboxyl group, O-glycosides, and N-glycosides. Derivatives of dextran can be obtained using known methods and materials. Derivatives of dextran are preferably used at or near neutrality.

The molecular weight of the dextran or a derivative thereof to be used in the transparentizing method of this aspect is not particularly limited. For example, the molecular weight may be 10 kDa or more, such as 20 kDa or more, 40 kDa or more, 60 kDa or more, 100 kDa or more, 150 kDa or more, or 200 kDa or more, and may be, for example, 10 kDa - 40 kDa, 10 kDa - 60 kDa, 10 kDa - 100 kDa, 10 kDa - 150 kDa, 10 kDa - 200 kDa, 20 kDa - 60 kDa, 20 kDa - 100 kDa, 20 kDa - 150 kDa, 20 kDa - 200 kDa, 40 kDa - 100 kDa, 40 kDa - 150 kDa, 40 kDa - 200 kDa, 60 kDa - 150 kDa, 60 kDa - 200 kDa, and 100 kDa - 200 kDa.

The solution comprising the dextran or a derivative thereof to be used in the transparentizing method of this aspect is preferably a solution in which the dextran or a derivative thereof is dissolved in an aqueous medium. Examples of the aqueous medium include water, a buffer solution, and a medium. The concentration of the dextran or a derivative thereof in the solution to be used in the method of this aspect is preferably such that the refractive index of the solution is about 1.35 or more, and preferably about 1.4 or more. The selection of the compound and the adjustment of its concentration are within the scope of those skilled in the art. Means and methods for measuring the refractive index are known.

The solution comprising the dextran or a derivative thereof to be used in the transparentizing method of this aspect may be a solution in which only the dextran or a derivative thereof is dissolved in an aqueous medium, and may contain a known transparentizing agent such as RapiClear, nucleotides, and the like, in addition to the dextran or a derivative thereof.

As another explanation on the transparentizing method of this aspect, the explanation on the transparentizing method using the compound of the present invention described above can be applied.

In a further aspect, the present invention provides the following:
a composition for making biological tissue transparent in a living state, comprising dextran or a derivative thereof;
a kit for making biological tissue transparent in a living state, comprising dextran or a derivative thereof;
a method for observing biological tissue, comprising making the biological tissue transparent in a living state by using the method, the composition, or the kit of the above aspect, and observing the inside of the biological tissue; and
a method for producing biological tissue made transparent in a living state, comprising applying the method, the composition, or the kit of the above aspect to the biological tissue.

As the methods for observing these composition, kit, and biological tissue and the method for producing the biological tissue, the explanation on the method for observing the composition, kit, and biological tissue and the method for producing the biological tissue described above can be applied.

The meanings of the terms used herein are to be understood as being normally understood in the fields of chemistry, biology, pharmacology, medicine and the like, unless otherwise stated.

The present invention will be explained in more detail and specifically below with reference to Examples; however, the scope of the present invention is not limited to Examples.

### Example 1

### (1) Permeability of Dextran, Polylysine, and Polyglutamic Acid into Collagen Gel

The permeability of FITC-α-polylysine (FITC-PLL) (15 kDa - 30 kDa), FITC-dextran (40 kDa), and FGApolyglutamic acid (FGA-PGA) (15 kDa - 30 kDa) into collagen gels was examined.

Collagen gels were used as a biological tissue model. Collagen gels and one of the above water-soluble polymers (1 mg/mL) were added to BPS (pH 7.4), and after 24 hours, the collagen gels were homogenized and centrifuged to measure the fluorescence of the resulting supernatant. The amount of polymer permeated into the gel was calculated from the fluorescence intensity. The results are shown in Figure 1. It was revealed that PGA and dextran permeate well into the collagen gel; whereas PLL is less likely to permeate into the collagen gel. These results indicated that the charge of the polymer transparentizing agent is important for permeating collagen gel and making it transparent, and that it is important for the charge to be at or near neutrality.

### (2) Impact of Molecular Weight of Dextran on Permeability into Collagen Gel

Using FITC-dextran (10 kDa, 40 kDa, 70 kDa, and 150 kDa), an experiment was conducted in the same manner as in Test Example 1. The results are shown in Fig. 2. It was revealed that the molecular weight of dextran does not affect the permeability into collagen gel.

### (3) Viability of Cells When Dextran, Polylysine, and Polyglutamic acid are Applied

PLL (1 kDa - 5 kDa) (380 mg/mL in DMEM), PGA (15 kDa - 50 kDa) (400 mg/mL in DMEM), or dextran (10 kDa, 60 kDa, and 200 kDa) (500 mg/mL in DMEM) was added to human dermal fibroblasts (1.0 × 10⁵ cells) seeded on a 24-well plate, and after incubation at 37°C for 90 minutes, the cell viability was measured using the WST-8 method. The results are shown in Figure 3. The cell viability with dextran of 10 kDa and 60 kDa was high. No significant decrease in viability was observed even with dextran of 200 kDa. On the other hand, the cell viability was low with PLL and PGA.

### (4) Impact of Dextran Concentration on Transparency of Collagen Gel

To collagen gel (type-I collagen (in PBS), 1.0% glutaraldehyde, refractive index of 1.41) placed on the bottom of a well of a 96-well plate, 200 µL of 10 kDa dextran solution (33, 50, or 60% w/w) was added, and after incubation at 37°C for 24 hours, the light transmittance at a wavelength of 600 nm was measured. The results are shown in Figure 4. As the dextran concentration increased, the transmittance increased, reaching 15% for 33% w/w dextran (refractive index of 1.38) and 34% for 50% w/w dextran (refractive index of 1.41).

The above results on transmittance and cell viability revealed that dextran has high cytocompatibility (low cytotoxicity) and also high transmittance through biological tissue.

### Example 2

### Making Biological Tissue Transparent Using Dextran

Using dextran, spheroids prepared using mouse L929 fibroblasts were made transparent.

The L929 cells (5 × 10⁵ cells) were cultured in DMEM for 7 days. A 96-well plate was used for the culture. The medium was replaced every day during the culture period. After 7 days of culture, the medium was aspirated and washed twice with PBS. To the spheroids thus obtained, 150 µL of DMEM (transparent medium), RapiClear (RC152001, SJL), or a dextran solution was added, and after incubation for 90 minutes, the cells were observed and photographed for transparency. The dextran solution was prepared by dissolving dextran (Dextran 40,000, FUJIFILM Wako Pure Chemical Corporation) in DEME (transparent medium) to make it 1000 mg/mL. The results are shown in Figure 4. It was found that dextran has transparentizing ability equivalent to that of the commercially available transparentizing drug RapiClear.

### Example 3

### Deep Observation of Three-Dimensional Tissue Using Dextran

### (1) Experimental Method

### (i) Experimental Procedure

1. Human dermal fibroblasts were seeded in a 10-cm² dish (1.5 × 10⁵ cells/mL), and left to stand for 1 day.
2. The medium was aspirated and washed with PBS, and then removed by aspiration.
3. After washing with PBS, 10 mL of Cell Tracker (trademark)/Medium was added thereto, and the mixture was left to stand for 1 hour.
4. After washing with PBS, 5 mL of trypsin was added thereto, and the mixture was left to stand for 5 minutes.
5. DMEM was added thereto, 15 ml of them was collected in a centrifugation tube and centrifuged at 1000 rpm for 5 minutes.
6. The supernatant was removed, and 1 ml of medium was added for pipetting.
7. 10 µL of cell dispersion and 10 µL of trypan blue solution were mixed by pipetting, and then the number of cells was counted.
8. 1 mL of 2% w/w collagen solution was added to 3 × 10⁶ cells, and the mixture was dispersed by pipetting.
9. 50 µL of dispersion solution was added dropwise onto a 35-mm dish and left to stand for 1 hour.
10. 2 mL of medium was added thereto, and the mixture was left to stand at 37°C for 1 day.
11. 2 mL of 50% w/w dextran (10 kDa) was added dropwise, and the mixture was incubated at 37°C for 90 minutes.
12. The three-dimensional tissue made transparent was photographed.
13. Deep observation was performed with a confocal quantitative image cytometer CQ-1 (Yokogawa Electric Corporation).

### (ii) Procedure for Preparing Fluorescent Reagent

1. 36 µL of DMSO was added to 50 µg of Cell Tracker (trademark) red to prepare 10 mM of Cell Tracker/DMSO.
2. 20 µL of Cell Tracker (trademark)/DMSO was added to 10 mL of phenol red-free medium to prepare 20 µM of Cell Tracker (trademark)/Medium.

### (2) Experimental Results

It was confirmed that the addition of dextran made the three-dimensional tissue (spheroid) transparent (Figure 5, lower left panel). Compared to the tissue without dextran, that with dextran had a larger area where cells were visible at any position of 100, 200, and 300 µm from the bottom (2 times, 2.2 times, and 6.2 times at 100 µm, 200 µm, and 300 µm from the bottom, respectively) (Figure 5, lower left panel). These results indicated that the use of dextran made the three-dimensional tissue transparent deep inside.

### Example 4

### Impact of Dextran on Viability of Cells in Three-Dimensional Tissue

### (1) Experimental Method

### (i) Experimental Procedure

1. Human dermal fibroblasts were seeded in a 10-cm² dish (1.5 × 10⁵ cells/mL), and left to stand for 1 day.
2. The medium was aspirated and washed with PBS, and then 5 mL of trypsin was added, and the mixture was left to stand for 5 minutes.
3. DMEM was added thereto, 15 ml of them was collected in a centrifugation tube and centrifuged at 1000 rpm for 5 minutes.
4. The supernatant was removed, and 1 ml of medium was added for pipetting.
5. 10 µL of cell dispersion and 10 µL of trypan blue solution were mixed by pipetting, and then the number of cells was counted.
6. 1 mL of 1% w/w collagen solution was added to 3 × 10⁶ cells, and the mixture was dispersed by pipetting.
7. 50 µL of dispersion solution was added dropwise onto a 35-mm dish and left to stand for 1 hour.
8. 2 mL of medium was added thereto, and the mixture was left to stand for 1 day.
9. 2 mL of Live dead reagent was added dropwise, and the mixture was left to stand at 37°C for 1 hour.
10. The Live dead reagent was aspirated, 2 mL of 50% w/w dextran (10 kDa) was added thereto, and the mixture was left to stand at 37°C for 90 minutes.
11. Deep observation was performed with CQ1.

### (ii) Procedure for Preparing Live dead Reagent

To 2 mL of transparent DMEM, 2 µL of calcein and 8 µL of Ethidium Homodimer-1 was mixed.

### (2) Experimental Results

The viability of the cells in the three-dimensional tissue was as high as 72% when the viability of that without dextran was taken as 100%. Further, the viability of the cells in the two-dimensional tissue was 72% when the viability of that without dextran was taken as 100%. It was found that, when the three-dimensional tissue was made transparent using dextran, the cell viability equivalent to that of the two-dimensional tissue was obtained. These results indicated that the use of dextran made the three-dimensional tissue transparent since dextran had sufficient cytocompatibility.

### Example 5

### Transparentizing Using Dextran Solution with AMP Added

### (1) Experimental Method

### (i) Preparation of Dextran Solution (Dex)

1000 mg of dextran (10 k, Sigma Aldrich Co. LLC, D9260) was dissolved in 1 mL of ultra pure water.

### (ii) Preparation of Dextran Solution supplemented with AMP (Dex + AMP)

1000 mg of dextran (10 k, Sigma Aldrich Co. LLC, D9260) and 500 mg of AMP (Sigma Aldrich Co. LLC, A1752) were dissolved in 1 mL of ultra pure water.

### (iii) Production of Collagen

1. 100 mg collagen powder (Nippi, PSC-1-200-500PW) was dissolved in 5 mL of PBS (concentration of × 1), and the mixture was homogenized for 5 minutes.
2. The mixture was left to stand overnight at 4°C to be thoroughly dissolved.
3. Centrifugation was performed (10000 rpm for 5 minutes) before use to remove bubbles.
4. The 2% (w/w) collagen solution obtained in 3. was subjected to gelation at 37°C.
5. The collagen obtained in 4. was cross-linked with 1% glutaraldehyde solution.

### (iv) Transparentizing Experiment

1. The cross-linked collagen gel obtained in (iii) was placed in a 96-well plate, and 200 µL of Dex solution or Dex + AMP solution was added per well.
2. The mixture was incubated at 37°C, and the transmittance was measured after a predetermined time using a plate reader (wavelength of 600 nm).

### (2) Experimental Results

The transmittance increased over time, and the transparency proceeded both the cases of using the Dex solution and the Dex + AMP solution. When the Dex + AMP solution was used, the transparency occurred more quickly, and the transmittance tended to be higher (Figure 8).

### Industrial Applicability

The present invention can be used in the development of pharmaceuticals, medical and biological research, and the like.

## Claims

1. A method for making biological tissue transparent in a living state, comprising applying a solution comprising dextran or a derivative thereof to the biological tissue.

2. The solution according to claim 1, wherein the dextran or a derivative thereof is present at a concentration such that the refractive index of the solution is 1.35 or more.

3. A composition for making biological tissue transparent in a living state, comprising dextran or a derivative thereof.

4. A kit for making biological tissue transparent in a living state, comprising dextran or a derivative thereof.

5. A method for observing biological tissue, comprising making the biological tissue transparent in a living state by using dextran or a derivative thereof, the method according to claim 1 or 2, the composition according to claim 3, or the kit according to claim 4, and observing the inside of the biological tissue.

6. A method for producing biological tissue made transparent in a living state, comprising applying dextran or a derivative thereof, the method according to claim 1 or 2, the composition according to claim 3, or the kit according to claim 4 to the biological tissue.
